# EUROPEAN PATENT APPLICATION

(11) **EP 4 755 299 A1**
(43) Date of publication of application: **10.06.2026**
(21) Application number: 24217382.1
(22) Date of filing: 04.12.2024
(51) Int. Cl.: A61B 5/11, A61B 5/00

(54) **METHOD FOR OPERATING A WEARABLE CARDIAC MOTION MONITORING DEVICE**

(71) Applicant: Roche Diabetes Care GmbH, 68305 Mannheim (DE); Roche Diabetes Care, Inc., Indianapolis, IN 46250-1025 (US)
(72) Inventor: BERG, Max, Manheim (DE); LIMBURG, Bernhard, Mannheim (DE); WEHOWSKI, Frédéric, Mannheim (DE); SURRIDGE, Nigel, Indianapolis (US); WINKELNKEMPER, Momme, Berlin (DE); USNER, Marcel, Berlin (DE)
(74) Representative: Pfiz/Gauss Patentanwälte PartmbB

(57) **Abstract**

The invention relates to a computer implemented method for operating a wearable cardiac motion monitoring device (10) comprising at least one motion sensor, the method comprising: acquiring motion data (12) from the at least one motion sensor in a standard mode (14); screening (32) the acquired motion data (12) for one or more predefined motion patterns (22, 24, 26) associated with a high accuracy mode (16, 16'); upon detecting a predefined motion pattern (22, 24, 26) in the acquired motion data (12), temporarily switching (28) to the high accuracy mode. The invention also relates to a corresponding wearable cardiac motion monitoring device.

## Description

### Field of the Invention

The invention relates to methods for operating a wearable cardiac motion monitoring device comprising at least one motion sensor and a corresponding device. The methods and corresponding device rely on a sensor data aggregation scheme that uses a standard mode and a high accuracy mode to save memory space and energy and increase the lifetime of the wearable cardiac motion monitoring device.

### Background of the Invention

For health care monitoring in patients wearable devices can be used. These devices are worn on the body to monitor the wearer's health data. They use biosensors or human body sensors to collect different data from the patient, such as heart rate, blood pressure, glucose levels, sleeping patterns, activity levels, etc. The collected data can be analyzed for correlations between different medical values, e.g., heart rate and activity patterns. They can be used to improve lifestyle or to signal emergencies.

A wearable cardiac motion monitoring device receives, stores and processes data requiring memory space and energy. To allow for high accuracy and high precision analyses of the sensor data, high accuracy sensor data, e.g., high frequency sensor data or data with a high resolution, must be stored and processed. However, memory space and energy are both limited resources on wearable devices. In addition, extensive use of the device reduces its lifetime. Therefore, the use of measuring devices in different measurement modes has been disclosed before.

US 10631739 B2 discloses a vital signs monitor that runs in a low power mode and in a high accuracy mode. In the low power mode movement is sampled at a lower frequency than in the high accuracy mode. The high accuracy mode is entered according to a schedule and/or in response to a corresponding command and/or in response to a wearer's monitored temperature falling below a threshold. In the high accuracy mode movement samples are stored at the vital signs monitor or transmitted. From the movement samples in the low power mode an activity level is determined, and the vital signs monitor is prevented from entering the high accuracy mode if the activity level exceeds a threshold. Thus, movement samples are only stored in high accuracy mode in case of a low activity level, e.g., if the safety of the user may be at risk.

Triggering a high accuracy mode only in case of a low activity level saves energy and memory space. It is an objective of the invention to save even more energy and memory space and to increase the lifetime of the cardiac motion monitoring device. In addition, high accuracy mode measurements may also be interesting in other situations that are not associated with a low activity level, e.g., when doing sports, etc. Therefore, it is another objective of the invention to allow for a more targeted operation of the wearable cardiac motion monitoring device.

The objectives are achieved by the invention specified in the independent claims. Advantageous embodiments and further developments of the invention are specified in the dependent claims.

### Summary of the invention

Embodiments of the invention concern methods for operating a wearable cardiac motion monitoring device that comprises at least one motion sensor and a corresponding device.

A first embodiment involves a method for operating a wearable cardiac motion monitoring device comprising at least one motion sensor, whose settings are defined by selecting one out of several motion data acquisition settings, one of the motion data acquisition settings allowing for acquiring motion data in a standard mode, and one or more of the motion data acquisition settings allowing for acquiring motion data with an increased accuracy in one or more high accuracy modes, the method comprising: acquiring motion data from the at least one motion sensor in the standard mode; screening the acquired motion data for one or more predefined motion patterns, wherein each of the predefined motion patterns is associated with a high accuracy mode; and, upon detecting a predefined motion pattern in the acquired motion data, temporarily selecting the motion data acquisition setting of the associated high accuracy mode .

A motion data acquisition setting defines one or more settings of the at least one motion sensor. It can comprise one or more of the group comprising the number of motion sensors, the type of one or more motion sensors, the number of channels of one or more motion sensors, the resolution of one or more motion sensors, the acquisition frequency of one or more motion sensors and postprocessing of motion data acquired by one or more motion sensors. By selecting a motion data acquisition setting, the at least one motion sensor is set to the settings defined by the motion data acquisition setting.

A motion data acquisition setting in standard mode is defined to acquire motion data at a lower level of accuracy than in a high accuracy mode. There may be two or more motion data acquisition settings in standard mode. Since the wearable cardiac motion monitoring device only switches to a high accuracy mode upon detection of a specific motion pattern, a more targeted operation of the device is possible. In this way, even more memory space (working memory or permanent storage) and energy are saved.

A motion data acquisition setting comprises one or more settings of one or more motion sensors. It increases the accuracy of the acquired motion data, if more motion data is acquired or if motion data of higher quality is acquired. More motion data is acquired, for example, if the number of used motion sensors is increased (e.g., an accelerometer and a gyroscope), if the resolution or the number of channels of a motion sensor is increased, or if the acquisition frequency is increased. Motion data of higher quality is acquired, for example, if a motion sensor of higher quality is used instead of a motion sensor of lower quality, e.g., if the noise is reduced, if a signal-to-noise ratio is increased, etc., or if the quality is improved by postprocessing of the acquired motion data.

A wearable cardiac motion monitoring device acquires cardiac motion information from a user. It can comprise, for example, a wristband, a chest band or a patch.

The at least one motion sensor can comprise an accelerometer and/or a gyroscope.

A motion pattern refers to a type of movement or to a sequence of movements such as "lying", "walking", "sleeping", etc.

The method can further comprise switching back from the high accuracy mode to the standard mode by selecting the motion data acquisition setting of the standard mode after a predefined period of time. The automatic leaving of a high accuracy mode to switch back to the standard mode saves memory space and energy.

The method can further comprise switching back from the high accuracy mode to the standard mode by selecting the motion data acquisition settings of the standard mode, when the acquired motion data does not belong to the one or more predefined motion patterns. In this way, motion data acquisition is terminated if the motion pattern no longer belongs to the predefined motion patterns, e.g., if the motion pattern is not of interest or disturbs the motion data acquisition process. In this way, the acquisition of not required or useless motion data as well as unsuccessful high accuracy mode measurements are prevented and, thus, memory space and energy are saved.

The method can further comprise switching back from the high accuracy mode to the standard mode by selecting the motion data acquisition setting of the standard mode upon receiving a user input. In this way, the user can prevent unnecessary motion data acquisition, thus saving memory space and energy. In addition, the user can exercise a more targeted operation of the device.

The method can further comprise switching back from the high accuracy mode to the standard mode by selecting the motion data acquisition setting of the standard mode upon receiving a signal from another device, e.g., from a smartphone or smartwatch, etc. In this way, the acquisition of motion data in a high accuracy mode is terminated upon a user request or in case the other device signals a change of motion pattern, etc. Thus, memory space and energy are saved, and a more targeted operation of the device is made possible.

According to an aspect of the invention, the acquired motion data is written to memory or transmitted to another device for storing in the standard mode and in the one or more high accuracy modes. By saving motion data during standard mode, contextual data is made available that can be used to better understand the motion data saved in high accuracy mode, e.g., which motion pattern led to the switch to the high accuracy mode, etc. In this way, a more thorough analysis of the motion data saved to memory is made possible. In addition, error handling and improvement of the scheme of switching between modes is made possible.

The one or more predefined motion patterns can be selected or modified with respect to different conditions. According to an example, the one or more predefined motion patterns depend on a temporal condition, e.g., on the current time, or on a history of detected predefined motion patterns. In this way, a more targeted and specified operation of the different modes of the device is possible. In addition, less useful or less interesting switches to high accuracy mode can be prevented and, thus, memory space and energy are saved.

To prevent saving less useful motion data to memory, a likelihood that a high accuracy mode measurement will be successful is determined from the acquired motion data, and the switching to the high accuracy mode is only carried out if the likelihood is above a threshold. In this way, memory space and energy are saved.

The likelihood that a high accuracy mode measurement will be successful can depend on at least one criterion from the group comprising a quality measure of the acquired motion data, the detected predefined motion pattern, a history of detected predefined motion patterns, a temporal condition.

The likelihood that a high accuracy mode measurement will be successful can also be determined using a trained machine learning model, wherein the trained machine learning model uses the acquired motion data and/or at least one criterion from the group comprising a quality measure of the acquired motion data, the detected predefined motion pattern, a history of detected predefined motion patterns, a temporal condition as input that is mapped to a likelihood of a successful high accuracy mode measurement as output. By using a machine learning model, the likelihood of a successful high accuracy mode measurement can be determined automatically and with a high accuracy, as the likelihood is learned from training data by optimizing some optimality criterion. The accurate likelihood saves memory and energy, and the machine learning model reduces user effort.

In an example, the predefined motion patterns are associated with at least two high accuracy modes with different motion data acquisition settings. By using two or more different high accuracy modes, the flexibility of the mode switching scheme is increased and, thus, a more targeted operation is made possible.

A wearable cardiac motion monitoring device according to a second embodiment comprises at least one motion sensor, whose settings are defined by selecting one out of several motion data acquisition settings, one of the motion data acquisition settings allowing for acquiring motion data in a standard mode, and one or more of the motion data acquisition settings allowing for acquiring motion data with an increased accuracy in one or more high accuracy modes, characterized by at least one processor comprising means configured to carry out a method according to the first embodiment of the invention.

A wearable cardiac motion monitoring device according to a third embodiment comprises at least one motion sensor, whose settings are defined by selecting one out of several motion data acquisition settings, one of the motion data acquisition settings allowing for acquiring motion data in a standard mode, and one or more of the motion data acquisition settings allowing for acquiring motion data with an increased accuracy in one or more high accuracy modes, and at least one processor configured for screening the acquired motion data for one or more predefined motion patterns, wherein each of the predefined motion patterns is associated with a high accuracy mode, and, upon detecting a predefined motion pattern in the acquired motion data, temporarily selecting the motion data acquisition setting of the associated high accuracy mode.

A computer program product according to a fourth embodiment of the invention comprises instructions which, when the program is executed by a processor, cause the processor to carry out a method according to the first embodiment of the invention.

A non-transitory computer-readable medium according to a fifth embodiment of the invention comprises a stored computer program executable by a computing device, the computer program comprising code for executing a method according to the first embodiment of the invention.

The invention described by examples and embodiments is not limited to the embodiments and examples but can be implemented by those skilled in the art by various combinations or modifications thereof.

### Brief Description of the Drawings

- Fig. 1: shows a flowchart of the steps of the method for operating a wearable cardiac motion monitoring device; and
- Fig. 2: illustrates the flow of data in a method for operating a wearable cardiac motion monitoring device;
- Fig. 3: illustrates a screening of motion data received from an accelerometer to detect predefined motion patterns.

### Detailed Description

In the following, advantageous exemplary embodiments of the invention are described and schematically shown in the figures. Throughout the figures and the description, same reference numbers are used to describe same features or components.

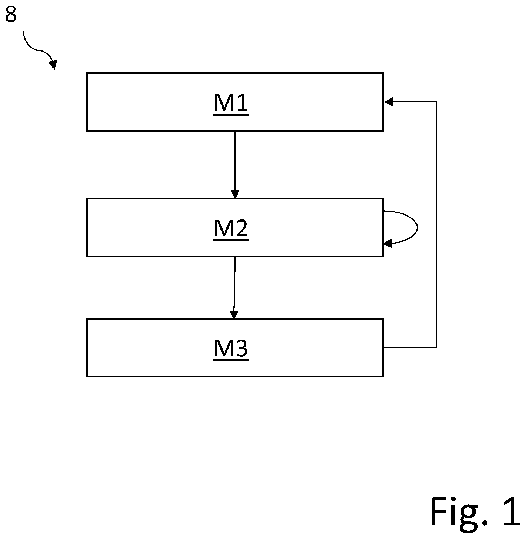

Fig. 1 illustrates a flow chart of a computer implemented method 8 for operating a wearable cardiac motion monitoring device comprising at least one motion sensor, whose settings are defined by selecting one out of several motion data acquisition setting, one of the motion data acquisition settings allowing for acquiring motion data in a standard mode, and one or more of the motion data acquisition settings allowing for acquiring motion data with an increased accuracy in one or more high accuracy modes, the method comprising: acquiring motion data from the at least one motion sensor in the standard mode in a step M 1; screening the acquired motion data for one or more predefined motion patterns, wherein each of the predefined motion patterns is associated with a high accuracy mode in a step M2; and, upon detecting a predefined motion pattern in the acquired motion data, temporarily selecting the motion data acquisition setting of the associated high accuracy mode in a step M3.

A high accuracy mode is only entered in case a specific motion pattern from a group of predefined motion patterns is detected. In this way, a more targeted operation of the wearable cardiac motion monitoring device is made possible. As the high accuracy mode is only entered on very specific conditions, the memory and energy consumption are further reduced.

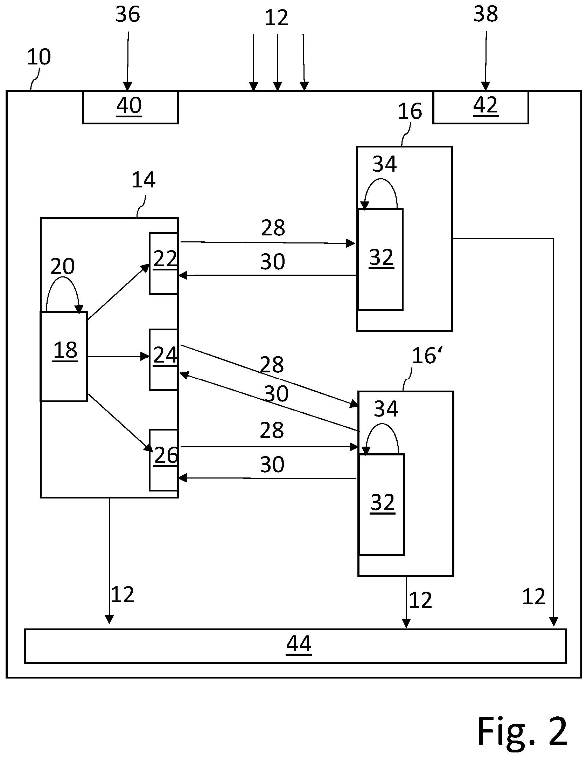

Fig. 2 illustrates the flow of data in a computer implemented method 8 for operating a wearable cardiac motion monitoring device 10 according to an embodiment of the invention. A wearable cardiac motion monitoring device can, for example, be a wristband, a ring, a chest band or a patch. The wearable cardiac motion monitoring device 10 acquires motion data 12 from at least one motion sensor. A motion sensor can measure acceleration in three spatial directions or angles or a higher derivative of velocity. For example, a motion sensor can comprise an accelerometer or a gyroscope. An accelerometer measures acceleration of an object. A gyroscope measures angular velocity and orientation. Using an accelerometer and a gyroscope in combination allows for measuring direction and length of movements.

The wearable cardiac motion monitoring device can be operated in a standard mode 14 or in a high accuracy mode 16, 16'. In Fig. 2, two high accuracy modes 16, 16' are shown. The standard mode 14 and the one or more high accuracy modes are each defined by a motion data acquisition setting defined by one or more settings of one or more motion sensors. A motion data acquisition setting provides information on, for example, which motion sensors are used, the number of motion sensors that are used, and specific settings of these motion sensors, e.g., a resolution of a motion sensor, a number of channels of a motion sensor, an acquisition frequency of a motion sensor, a postprocessing of the acquired motion data 12 of a motion sensor, etc.

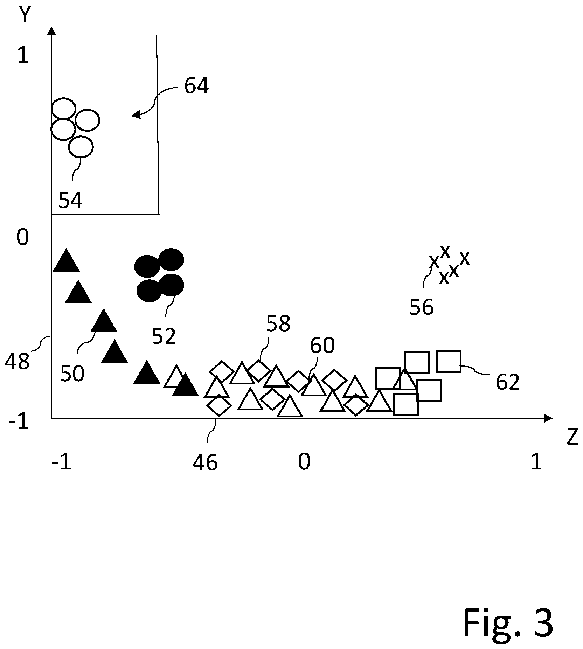

In standard mode 14, the motion data 12 is continuously 20 screened 18 for one or more predefined motion patterns 22, 24, 26. The screening can be carried out as shown, for example, with respect to Fig. 3 below. A motion data acquisition setting in standard mode 14 is defined to save less motion data to memory in order to save memory and/or energy but still allow for a detection of predefined motion patterns. In case one of the one or more predefined motion patterns 22, 24, 26 is detected, the wearable cardiac motion monitoring device 20 switches 28 from standard mode 14 to the high accuracy mode 16, 16' that is associated with the detected motion pattern 22, 24, 26 by selecting the associated motion data acquisition setting that defines settings of the at least one motion sensor. The at least one motion sensor is, thus, set to the settings defined by the motion data acquisition setting associated with the high accuracy mode 16, 16'. Two or more motion patterns 22, 24, 26 can be associated with the same high accuracy mode 16, 16'. In this case, motion pattern 22 is associated with a first high accuracy mode 16, while motion patterns 24, 26 are associated with a second high accuracy mode 16'. A first high accuracy mode 16 can, for example, be used for ECG measurements during resting, a second high accuracy mode can, for example, be used for ECG measurements during sports, etc. In an example, the predefined motion patterns 22, 24, 26 are associated with at least two high accuracy modes 16, 16' that are defined by different motion data acquisition settings. Thus, at least two different high accuracy modes 16, 16' exist.

A predefined motion pattern can include movements such as "sleeping", "lying in a supine position", "eating", "coughing", "walking", "running", "lying in a prone position", "standing", "sitting", "lying in a lateral position", etc. A predefined motion pattern can include a sequence of movements, e.g., "lying in a supine position" followed by "rising up the upper body" followed by "coughing", or "walking" followed by "eating" followed by "walking".

As the heart rate can best be analyzed during sleeping without interfering movements, "lying still" for a certain period of time or "sleeping" can, for example, trigger a high accuracy mode.

The predefined motion patterns 22, 24, 26 can be changed depending on various conditions. In an example, the predefined motion patterns 22, 24, 26 depend on a temporal condition, e.g., on the current time, on the time of day, on the day of the week or on the current season, etc. For example, different predefined motion patterns can trigger a high accuracy mode 16, 16' by day or by night, e.g., "coughing" at night can trigger a high accuracy mode, whereas "coughing" by day does not trigger a high accuracy mode, or "walking" on a weekday can trigger a high accuracy mode, whereas "walking" on weekends does not trigger a high accuracy mode, etc.

In an example, the predefined motion patterns 22, 24, 26 depend on a history of detected predefined motion patterns. Thus, motion patterns may be more interesting for analysis in case of specific preceding motion patterns. For example, "coughing" after "running" may trigger a high accuracy mode 16, 16', whereas "coughing" after "sleeping" may not trigger a high accuracy mode 16, 16'.

In an example, a likelihood that a high accuracy mode measurement will be successful is determined from the acquired motion data 12, and the switching 28 to the high accuracy mode 16, 16' is only carried out if the likelihood is above a threshold. Thus, the motion data acquisition settings of the high accuracy mode 16, 16' are only selected if the likelihood is above the threshold. Different conditions can apply to obtain a successful high accuracy mode measurement. The likelihood that a high accuracy mode measurement will be successful can, for example, depend on at least one criterion from the group comprising a quality measure of the acquired motion data 12, the detected predefined motion pattern 22, 24, 26, a history of detected predefined motion patterns 22, 24, 26, a temporal condition. In particular, the likelihood that a high accuracy mode measurement will be successful depends on at least one criterion from the group consisting of a quality measure of the acquired motion data, the detected predefined motion pattern, a history of detected predefined motion patterns, a temporal condition.

A quality measure of the acquired motion data determines the suitability of the acquired motion data for obtaining high accuracy mode measurement for further analyses.

In an example, a quality measure is defined with respect to the number of measurements acquired during the same motion pattern, or during a group of motion patterns or during a specific period of time. For example, the more measurements are acquired the higher is the likelihood of a successful high accuracy mode measurement. In another example, a high accuracy mode measurement may only be possible if a minimum number of measurements is acquired during the same motion pattern, the same group of motion patterns or during a specific period of time.

In an example, a quality measure is defined with respect to a duration of a detected predefined motion pattern. For example, the longer a motion pattern takes, the higher is the likelihood of a successful high accuracy mode measurement. In another example, a high accuracy mode measurement may only be possible if a motion pattern takes a minimum amount of time, e.g., measuring a resting heart rate.

In an example, a quality measure is defined with respect to the acquired motion data itself, e.g., with respect to a noise level of the motion data, with respect to a signal-to-noise ratio of the motion data, with respect to the motion data lying within a specific range or interval, with respect to the strength or the frequency of variations in the motion data, etc.

The likelihood that a high accuracy mode measurement will be successful can depend on the detected predefined motion pattern. To this end, a motion pattern, i.e., a movement or a sequence of movements, can be associated with a likelihood that a high accuracy mode measurement will be successful. For example, a high accuracy mode measurement of a heart rate will be less likely during "coughing" than during "sleeping".

The likelihood that a high accuracy mode measurement will be successful can depend on a history of detected predefined motion patterns. For example, a sequence of "running" followed by "lying" will probably not lead to a successful measurement of a resting heart rate or a long-term heart rate and will, thus, be associated with a low likelihood.

The likelihood that a high accuracy mode measurement will be successful can depend on a temporal condition, e.g., on the current time, on the time of day, on the day of the week, on the current season, etc. For example, "sleeping" by day may have a lower likelihood of producing a successful high accuracy mode measurement than "sleeping" by night, since "sleeping" is a motion pattern that is usually carried out for longer periods of time and with a lower likelihood of being disturbed during night than during day.

The likelihood that a high accuracy mode measurement will be successful can depend on further conditions, e.g., on properties or of a user or user habits.

A likelihood that a high accuracy mode measurement will be successful can be determined using a trained machine learning model. The trained machine learning model uses the acquired motion data and/or at least one criterion from the group comprising a quality measure of the acquired motion data, the detected predefined motion pattern, a history of detected predefined motion patterns, a temporal condition as input that is mapped to a likelihood of a successful high accuracy mode measurement as output. By use of a machine learning model, a likelihood for a successful high accuracy mode measurement can be derived specifically for each user and his specific user habits. In addition, the machine learning model learns automatically from training data, thereby optimizing an objective function, instead of using suboptimal rules defined by a human to determine a likelihood of a successful high accuracy mode measurement.

To train the machine learning model, training data can be collected containing input data such as a sequence of acquired motion data, a sequence of detected predefined motion patterns, temporal information, properties of the user, etc., and corresponding outputs indicating if a following high accuracy mode measurement was successful or not. In an example, a sequence comprising a sequence of, e.g., 10 or more, previously acquired motion data samples together with the time of day and the three previously detected predefined motion patterns can be used as input. Alternatively, only a sequence of, e.g., 10 or more, previously acquired motion data samples can be used as input. The machine learning model can, for example, be a deep learning neural network such as a multilayer perceptron, a convolutional neural network, a recurrent neural network, etc. The machine learning model contains a number of neurons corresponding to the size of the input as input layer and a single neuron indicating the likelihood of a successful high accuracy mode measurement as output. The machine learning model can be trained using a variant of the backpropagation algorithm known to a person skilled in the art.

The wearable cardiac motion monitoring device 10 only enters into a high accuracy mode 16, 16' if the likelihood of a successful high accuracy mode measurement lies above a threshold for the detected motion pattern. In this way, memory and energy consumption can be reduced further.

When switching 28 to a high accuracy mode 16, 16' one or more settings of the at least one motion sensor are modified depending on the motion data acquisition setting associated with the high accuracy mode 16, 16'. Each high accuracy mode 16, 16' is defined by a different motion data acquisition setting that increases the accuracy of the acquired motion data compared to the motion data acquisition setting in standard mode. Due to the increasing amount of collected motion data, the memory and/or energy consumption increase. A motion data acquisition setting comprises, for example, one or more of the group comprising the number of motion sensors, the type of one or more motion sensors, the number of channels of one or more motion sensors, the resolution of one or more motion sensors, the acquisition frequency of one or more motion sensors, postprocessing of the motion data acquired by one or more motion sensors, etc. In particular, a motion data acquisition setting can be defined by one or more of the group comprising the number of motion sensors, the type of one or more motion sensors, the number of channels of one or more motion sensors, the resolution of one or more motion sensors, the acquisition frequency of one or more motion sensors and postprocessing of motion data acquired by one or more motion sensors. In particular, a motion data acquisition setting can be defined by one or more of the group consisting of the number of motion sensors, the type of one or more motion sensors, the number of channels of one or more motion sensors, the resolution of one or more motion sensors, the acquisition frequency of one or more motion sensors and postprocessing of motion data acquired by one or more motion sensors.

By increasing the number of motion sensors whose motion data is acquired the accuracy of the acquired motion data is increased. For example, in standard mode 14 only accelerometer motion data is used, whereas in a high accuracy mode also gyroscope motion data is used.

By modifying the type of one or more motion sensors used for acquiring motion data, the accuracy of the acquired motion data 12 can be increased. For example, a motion sensor of low accuracy (e.g., of lower resolution, with a lower number of channels, of lower quality, with a higher noise level, etc.) can be used in standard mode 14, whereas a motion sensor of high accuracy (e.g., of higher resolution, with a higher number of channels, of higher quality, with a lower noise level, etc.) is used in a high accuracy mode 16, 16'.

By increasing the number of channels of one or more motion sensors more motion data is acquired. For example, when using an accelerometer to acquire motion data, motion in one spatial direction can be measured in standard mode, whereas motion in two or three spatial directions can be measured in a high accuracy mode. The increased number of channels allow the acquisition of more accurate information and, thus, a more detailed analysis of the acquired motion data 12.

By increasing the resolution of one or more motion sensors, more accurate information is acquired and used for further analysis. For example, the resolution of a camera can be increased, or the resolution of an accelerometer can be increased.

By increasing the acquisition frequency of one or more motion sensors, motion data is acquired more frequently at shorter time intervals. In his way, even short-time changes in the motion data are acquired. These allow for a more accurate analysis of the motion data.

By applying postprocessing to the motion data of one or more motion sensors, more accurate information can be extracted from the motion data. A lowpass filter can, for example, be used to reduce noise, a Kalman filter can be used to reduce outliers and noise in the measurements or to obtain meaningful motion patterns from the acquired motion data 12. For example, only specific frequency ranges of interest can be analyzed in the motion data, e.g., by using Fourier transforms, etc.

In addition to detecting predefined motion patterns in the motion data 12 acquired by the at least one motion sensor, a high accuracy mode 16, 16' can be triggered by a user input 36 in case the user wants to manually initiate a measurement, e.g., by pressing a button or by making a specific movement, etc. When the wearable cardiac motion monitoring device 10 receives the user input 36, a manual trigger 40 can trigger one of the high accuracy modes 16, 16'. Which one of the high accuracy modes 16, 16' is triggered can be an information contained in the user input 36, e.g., by using a specific button or by entering a shortcut for the desired type of measurement. The type of measurement is then internally associated with a high accuracy mode 16, 16'.

In addition, a high accuracy mode 16, 16' can be triggered by a device signal 38, e.g., a signal received from another device such as a smartwatch, bracelet or smartphone, etc. For example, the other device can send a current motion pattern such as a "sleeping state" or "workout state" to the wearable cardiac motion monitoring device 10. An outside trigger 42 can screen the received motion patterns for one of the one or more predefined motion patterns 22, 24, 26. In case a received motion pattern belongs to one of the one or more predefined motion patterns, an outside trigger 42 can trigger the high accuracy mode 16, 16' associated with the received motion pattern.

There are different options for the wearable cardiac motion monitoring device 10 to switch back 30 from high accuracy mode 16, 16' to standard mode 14 by selecting the motion data acquisition setting of the standard mode 14 to define settings of the at least one motion sensor. For example, in high accuracy mode 16, 16' the motion data 12 acquired by the one or more motion sensors is continuously 34 screened 32 for the one or more predefined motion patterns 22, 24, 26. If the current motion pattern no longer belongs to the one or more predefined motion patterns 22, 24, 26, the wearable cardiac motion monitoring device 10 switches back 30 to standard mode 14. In addition or alternatively, the wearable cardiac motion monitoring device 10 can switch back 30 to standard mode 14 after a predefined period of time. The predefined period of time can be selected to allow for an acquisition of sufficient motion data 12 for a further analysis, but at the same time to limit the acquisition in high accuracy mode 16, 16' to the required amount of motion data in order to save memory space and energy. In addition or alternatively, the wearable cardiac motion monitoring device 10 can switch back 30 to standard mode 14 upon a user input 36 or upon a device signal 38, e.g., indicating that the current motion pattern has ended, or if the current motion pattern no longer belongs to the one or more predefined motion patterns 22, 24, 26.

The acquired motion data 12 can be written to memory 44, e.g., to a working memory or to a permanent storage, or it can be transmitted to another device for storing. The writing to memory 44 can be carried out in at least one of the high accuracy modes 16, 16'. The writing to memory 44 can be carried out in all high accuracy modes 16, 16'. The writing to memory 44 can be carried out in standard mode 14. In an example, the acquired motion data 12 is written to memory in standard mode 14 and in the one or more high accuracy modes 16, 16'. The acquired motion data 12 written to memory 44 in standard mode 14 may not suffice for a desired analysis, e.g., for a gyrocardiogram. Nevertheless, this motion data 12 can be used as contextual data, e.g., to obtain information on the motion pattern 22, 24, 26 that triggered the high accuracy mode 16, 16' or information on which motion data 12 preceded the switching 28 to the high accuracy mode 16, 16'. Such contextual data may be important for understanding or evaluating the motion data 12 written to memory 44 or transmitted to another device in a high accuracy mode 16, 16'.

Fig. 3 illustrates a screening of motion data 23 received from an accelerometer to detect predefined motion patterns 22, 24, 26. Specifically, the graphic shows a gyrocardiography measurement when lying in a supine position The horizontal axis 46 indicates a normalized movement in Z-direction, whereas the vertical axis 48 indicates a normalized movement in Y-direction. From the coordinates different motion patterns can be discriminated, e.g., "moving, eating, moving" 50, "lying in a lateral position" 52, "lying in a supine position" 54, "lying in a prone position" 56, "walking" 58, "sitting" 60, "standing" 62. Specific regions 64 can be associated with a predefined motion pattern 22, 24, 26, e.g., the region 64 defining the "lying in a supine position" 54 motion pattern. The regions 64 can, for example, be obtained using machine learning methods such as support vector machines, clustering methods (e.g., using the expectation maximization algorithm or k nearest neighbors), decision trees, random forests, classification neural networks, etc. Using machine learning methods, motion patterns can be automatically identified from the acquired motion data. Alternatively, the regions can be identified by a user.

The predefined motion patterns that are associated with a high accuracy mode can then be selected from the identified motion patterns, e.g., depending on the further analyses that are to be carried out using the stored motion data. For example, if a resting heart rate is to be measured, "sleeping" or "lying in a supine position" can be used as a predefined motion pattern that triggers a first high accuracy mode. The motion data stored during the first high accuracy mode can then be used for determining a resting heart rate. If a heart rate during workout is to be measured, "walking" can be used as a predefined motion pattern that triggers a second high accuracy mode. The motion data stored during the second high accuracy mode can then be used for determining a heart rate during workout.

Reference throughout this specification to "an embodiment" or "an example" or "an aspect" means that a particular feature, structure or characteristic described in connection with the embodiment, example or aspect is included in at least one embodiment, example or aspect. Thus, appearances of the phrases "according to an embodiment", "according to an example" or "according to an aspect" in various places throughout this specification are not necessarily all referring to the same embodiment, example or aspect, but may. Furthermore, the particular features or characteristics may be combined in any suitable manner, as would be apparent to one of ordinary skill in the art from this disclosure, in one or more embodiments.

Furthermore, while some embodiments, examples or aspects described herein include some but not other features included in other embodiments, examples or aspects combinations of features of different embodiments, examples or aspects are meant to be within the scope of the claims, and form different embodiments, as would be understood by those skilled in the art.

In summary, the invention relates to a computer implemented method for operating a wearable cardiac motion monitoring device 10 comprising at least one motion sensor, the method comprising: acquiring motion data 12 from the at least one motion sensor in a standard mode 14; screening 32 the acquired motion data 12 for one or more predefined motion patterns 22, 24, 26 associated with a high accuracy mode 16, 16'; upon detecting a predefined motion pattern 22, 24, 26 in the acquired motion data 12, temporarily switching 28 to the associated high accuracy mode 16, 16'. The invention also relates to a corresponding wearable cardiac motion monitoring device.

### Reference number list

- 8: Computer implemented method
- 10: Cardiac motion monitoring device
- 12: Motion data
- 14: Standard mode
- 16, 16': High accuracy mode
- 18: Screening
- 20: Continuous
- 22, 24, 26: Predefined motion pattern
- 28: Switch
- 30: Switch back
- 32: Screening
- 34: Continuous
- 36: User input
- 38: Device signal
- 40: Manual trigger
- 42: Outside trigger
- 44: Memory
- 46: Horizontal axis
- 48: Vertical axis
- 50: "Moving, eating, moving"
- 52: "Lying in a lateral position"
- 54: "Lying in a supine position"
- 56: "Lying in a prone position"
- 58: "Walking"
- 60: "Sitting"
- 62: "Standing"

## Claims

1. Computer implemented method (8) for operating a wearable cardiac motion monitoring device (10) comprising at least one motion sensor, whose settings are defined by selecting one out of several motion data acquisition settings, one of the motion data acquisition settings allowing for acquiring motion data (12) in a standard mode (14), and one or more of the motion data acquisition settings allowing for acquiring motion data (12) with an increased accuracy in one or more high accuracy modes (16, 16'), the method comprising:
- Acquiring motion data (12) from the at least one motion sensor in the standard mode (14);
**characterized by**:
- Screening (32) the acquired motion data (12) for one or more predefined motion patterns (22, 24, 26), wherein each of the predefined motion patterns (22, 24, 26) is associated with a high accuracy mode (16, 16');
- Upon detecting a predefined motion pattern (22, 24, 26) in the acquired motion data (12), temporarily switching (28) to the associated high accuracy mode (16, 16') by selecting the motion data acquisition setting of the associated high accuracy mode (16, 16').

2. The method of claim 1, wherein a motion data acquisition setting comprises one or more of the group comprising the number of motion sensors, the type of one or more motion sensors, the number of channels of one or more motion sensors, the resolution of one or more motion sensors, the acquisition frequency of one or more motion sensors, postprocessing of the motion data (12) acquired by one or more motion sensors.

3. The method of any one of the preceding claims, further comprising switching back (30) from the high accuracy mode (16, 16') to the standard mode (14) by selecting the motion data acquisition setting of the standard mode (14) after a predefined period of time.

4. The method of any one of the preceding claims, further comprising switching back (30) from the high accuracy mode (16, 16') to the standard mode (14) by selecting the motion data acquisition setting of the standard mode (14) when the acquired motion data (12) does not belong to the one or more predefined motion patterns (22, 24, 26).

5. The method of any one of the preceding claims, further comprising switching back (30) from the high accuracy mode (16, 16') to the standard mode (14) by selecting the motion data acquisition setting of the standard mode (14) upon receiving a user input (36) or upon receiving a signal (38) from another device.

6. The method of any one of the preceding claims, wherein the acquired motion data (12) is written to memory (44) or transmitted to another device for storing in the standard mode (14) and in the one or more high accuracy modes (16, 16').

7. The method of any one of the preceding claims, wherein the one or more predefined motion patterns (22, 24, 26) depend on a temporal condition.

8. The method of any one of the preceding claims, wherein the one or more predefined motion patterns (22, 24, 26) depend on a history of detected predefined motion patterns.

9. The method of any one of the preceding claims, wherein a likelihood that a high accuracy mode measurement will be successful is determined from the acquired motion data (12), and wherein the switching (28) to the high accuracy mode (16, 16') is only carried out if the likelihood is above a threshold.

10. The method of claim 9, wherein the likelihood that a high accuracy mode measurement will be successful depends on at least one criterion from the group comprising a quality measure of the acquired motion data (12), the detected predefined motion pattern (22, 24, 26), a history of detected predefined motion patterns (22, 24, 26), a temporal condition.

11. The method of claim 9 or 10, wherein the likelihood that a high accuracy mode measurement will be successful is determined using a trained machine learning model, wherein the trained machine learning model uses the acquired motion data (12) and/or at least one criterion from the group comprising a quality measure of the acquired motion data (12), the detected predefined motion pattern (22, 24, 26), a history of detected predefined motion patterns (22, 24, 26), a temporal condition as input that is mapped to a likelihood of a successful high accuracy mode measurement as output.

12. The method of any one of the preceding claims, wherein the predefined motion patterns (22, 24, 26) are associated with at least two high accuracy modes (16, 16') with different motion data acquisition settings.

13. The method of any one of the preceding claims, wherein the at least one motion sensor comprises an accelerometer and/or a gyroscope.

14. A computer program product comprising instructions which, when the program is executed by a processor, cause the processor to carry out a method according to any one of the preceding claims.

15. A wearable cardiac motion monitoring device (10) comprising
- at least one motion sensor, whose settings are defined by selecting one out of several motion data acquisition settings, one of the motion data acquisition settings allowing for acquiring motion data (12) in a standard mode (14), and one or more of the motion data acquisition settings allowing for acquiring motion data (12) with an increased accuracy in one or more high accuracy modes (16, 16'), and
- at least one processor comprising means configured to carry out a method according to any one of claims 1 to 13.
